# EUROPÄISCHE PATENTSCHRIFT

(11) **EP 2 769 837 B1**
(45) Veröffentlichungstag und Bekanntmachung des Hinweises auf die Patenterteilung: **26.04.2017**
(21) Anmeldenummer: 14000635.4
(22) Anmeldetag: 24.02.2014
(51) Int. Cl.: B31B 50/36, B31B 50/74, B31B 100/00, B31B 110/35, B31B 120/30

(54) **Verfahren und Vorrichtung zum Prüfen von Faltzuschnitten**
Method and device for inspecting folded blanks
Procédé et dispositif de contrôle de découpes de pliage

(30) Priorität: 25.02.2013 DE 102013003090
(43) Veröffentlichungstag der Anmeldung: 27.08.2014
(73) Patentinhaber: Texmag GmbH Vertriebsgesellschaft, 8800 Thalwil (CH)
(72) Erfinder: Reiter, Thomas, 85235 Odelzhausen (DE); Zwerger, Lars, 86163 Augsburg (DE)
(74) Vertreter: Witzany, Manfred

(56) Entgegenhaltungen:
- DE-A1-102008 017 444
- US-A- 3 433 135

## Beschreibung

Die Erfindung betrifft ein Verfahren zum Prüfen von mindestens einem aus flächigem Material gebildeten Faltzuschnitt sowie eine Vorrichtung zur Durchführung dieses Verfahrens.

Aus der EP 2 141 487 A1 ist ein Inspektionssystem zur Erfassung von Schmutzstellen auf Kartons bekannt. Hierzu wird der Karton mit einer Kamera erfasst.

Aus der EP 0 611 608 A1 ist ein Verfahren zum dreidimensionalen Abtasten von Erhebungen eines Objekts bekannt. Hierbei wird ein erheblicher apparativer Aufwand getrieben. Außerdem ist die Auswertung der Messergebnisse sehr rechenaufwendig. Damit kann auf diese Weise nur eine relativ langsame Abtastung erfolgen, die für einen industriellen Produktionsprozess ungeeignet ist.

Aus der US 5,978,499 A ist eine Vorrichtung zur inspektion von Kartons bekannt, die die Außenkanten der Kartons mittels Bahnkantensensoren erfasst.

Aus der EP 0 330 495 A2 ist ein Inspektionssystem für Verpackungen bekannt, welches Zeilenkameras einsetzt. Auch mit diesem Inspektionssystem lassen sich nur die Außenkanten der Verpackungen erfassen.

Aus der EP 0 677 444 A1 ist ein optisches Kontrollverfahren bekannt. Dabei wird ein Objekt mit einer Kamera aufgenommen und mit einer Referenz verglichen. Aus Abweichungen zwischen dem aufgenommenen Ist-Bild und dem gespeicherten Soll-Bild werden fehlerhafte Objekte erkannt.

Aus der US 8,073,239 B1 ist eine Vorrichtung bekannt. In dieser Vorrichtung wird ein Wellpappen-Faltzuschnitt transportiert und gleichzeitig in Lagen übereinander gefaltet. Dabei werden die zusammengefalteten Enden mittels Klebelaschen miteinander verklebt. Dabei ist es wichtig, dass die Faltung und anschließende Verklebung präzise erfolgen. Zu diesem Zweck weist die Vorrichtung eine Lichtquelle und eine Kamera auf, die den Faltzuschnitt optisch nach dem Reflexionsprinzip abtasten. Häufig kommt es vor, dass der gefaltete und verklebte Faltzuschnitt mantelartig geformt ist. In diesem Fall liegt die verklebte Lage unmittelbar auf der darunter liegenden Lage auf. Dabei tastet das Reflexionsverfahren beide Lagen gemeinsam ab, so dass die für die Qualitätsbeurteilung interessierenden Randkanten des Faltzuschnittes nicht mit dem erforderlichen Kontrast ermittelbar sind. Damit ist dieses bekannte Verfahren auf die Anwendung von Sonderfällen eingeschränkt und kann nicht universell eingesetzt werden.

Aus der DE 10 2008 017 444 A1 ist eine Vorrichtung zur Herstellung von Säcken aus Schlauchstücken bekannt. Zur Überwachung der Geometrie des offenen Bodenrechtecks, der Eckeinschläge und des verschlossenen Bodens ist eine entsprechende Überwachungsvorrichtung vorgesehen. Diese weist Sensoren auf, die die Farbe, Reflektivität oder andere Eigenschaften erfasst und damit eine Kantenbestimmung ermöglichen.

Aus der US 3,433,135 A ist eine gattungsgemäße Vorrichtung zum Prüfen eines flächigen und in einer Laufrichtung transportierten Faltzuschnitts offenbart. Dieser Faltzuschnitt ist in Lagen gefaltet. Die Vorrichtung weist einen Emitter und einen Detektor sowie ein Schwert auf, welches zwischen den Lagen des Faltzuschnitts gehalten ist. Die Vorrichtung weist außerdem ein Bauteil auf, welches außerhalb der Lagen vorgesehen ist, und mit einem Emitter und einem Detektor ausgerüstet ist, um Lichtwellen auf den Faltzuschnitt bzw. das Schwert zu senden und von diesem reflektiertes Licht zu empfangen. Dabei weist das Schwert eine Reflektivität für das Licht auf, die sich von der Reflektivität des Faltzuschnitts unterscheidet, um auf diese Weise Faltzuschnittkanten zu ermitteln. Diese bekannte Vorrichtung hat sich in der Praxis bewährt und bildet den Ausgangspunkt der vorliegenden Erfindung.

Der Erfindung liegt die Aufgabe zugrunde, ein Verfahren sowie eine Vorrichtung zur Durchführung dieses Verfahrens der eingangs genannten Art zu schaffen, die bei unterschiedlichsten Anwendungsfällen universell einsetzbar sind.

Diese Aufgabe wird neuerungsgemäß mit den folgenden Merkmalen gelöst.

Das erfindungsgemäße Verfahren dient zum Prüfen von mindestens einem aus flächigem Material gebildeten Faltzuschnitt. Vorzugsweise besteht der Faltzuschnitt aus Karton oder Wellpappe, insbesondere zur Herstellung von Verpackungen. Das erfindungsgemäße Verfahren ist jedoch nicht auf diese Materialien beschränkt. Der mindestens eine Faltzuschnitt wird dabei in einer Laufrichtung transportiert, um vorzugsweise eine Vielzahl von Faltzuschnitten sequentiell herstellen zu können. Quer zur Laufrichtung wird der Faltzuschnitt in Lagen aufeinander gefaltet. Es ist insbesondere daran gedacht, diese aufeinander gefalteten Lagen miteinander zu verbinden, wobei hierfür unterschiedliche Verbindungsmittel wie beispielsweise Kleben, Klammern oder Nieten einsetzbar sind. Durch das Verbinden ergibt sich aus dem Faltzuschnitt ein rohrartiger Körper, der allerdings flach zusammengelegt ist. Der Faltzuschnitt selbst kann bedruckt oder unbedruckt sein. Es ist aus mehreren Gründen wichtig, dass diese Faltung exakt ist. Eine präzise Faltung erhöht die Stabilität, wenn der Faltzuschnitt letztlich in seine geometrische Form gefaltet wird. Außerdem neigen Faltmaschinen bei einer unpräzisen Faltung zu einem Materialstau. Insbesondere bei bedruckten Faltzuschnitten ist eine exakte Faltung zur Erzielung eines sauberen Druckbildes auch im Bereich der Verbindungsstelle wichtig. Dabei kommt es insbesondere darauf an, dass im Wesentlichen senkrecht zu der Verbindungsstelle verlaufende Randkanten des Faltzuschnittes möglichst genau fluchten. Anderenfalls ergäbe sich ein unerwünschter Versatz zwischen den Mantelflächen des Faltzuschnittes und ggf. ein entsprechender Versatz im Druckbild. Erschwerend kommt hinzu, dass diese Randkanten nicht immer gerade verlaufen. Andererseits ist auch die Einstellung der exakten Überlappungslänge wichtig, um beispielsweise eine möglichst exakte quaderförmige Form des entfalteten Faltzuschnitts zu gewährleisten und eine korrekte Spaltbreite einzustellen. Um dies zu gewährleisten, muss das erfindungsgemäße Verfahren entsprechende Ausrichtungsfehler zuverlässig erkennen können. Problematisch ist in diesem Zusammenhang allerdings, dass der Faltzuschnitt in der Regel in Lagen aufeinander gefaltet, also in flacher Konfiguration vorliegt. Es muss daher sichergestellt sein, dass bei diesem Verfahren Randkanten jener Lage des Faltzuschnittes erfasst werden, die während des Faltvorgangs umgelegt wurden. Dagegen muss die beim Faltvorgang unbewegte Lage möglichst von der Prüfung ausgeschlossen werden.

Zur Lösung dieses Problems erfolgt die Faltung der mindestens einen Lage auf mindestens ein Schwert, welches sich nach dem Faltvorgang somit zwischen den Lagen des mindestens einen Faltzuschnitts befindet. Diesem mindestens einen Schwert liegt mindestens ein Bauteil gegenüber, welches außerhalb des Faltzuschnitts vorgesehen ist. Auf diese Weise ist sichergestellt, dass sich zwischen dem mindestens einen Bauteil und dem mindestens einen Schwert lediglich die zu prüfende mindestens eine Lage befindet, welche beim Faltvorgang umgelegt wurde. Die beim Faltvorgang unbewegte, mindestens eine Lage wird dagegen durch das mindestens eine Schwert verdeckt. Zwischen dem mindestens einen Schwert und dem mindestens einen Bauteil werden Wellen propagiert, mit deren Hilfe die zwischen dem mindestens einen Schwert und dem mindestens einen Bauteil befindliche mindestens eine Lage abgetastet wird. Zu diesem Zweck weist das mindestens eine Schwert bezüglich der Wellen mindestens eine physikalische Eigenschaft auf, die sich von der entsprechenden physikalischen Eigenschaft des mindestens einen Faltzuschnitts unterscheidet. Auf diese Weise ist sichergestellt, dass zwischen dem mindestens einen Schwert und dem mindestens einen Bauteil ein Kontrast in der Wellenausbreitung erzielt wird, je nachdem ob die mindestens eine Lage des mindestens einen Faltzuschnitts zwischen dem mindestens einen Schwert und dem mindestens einen Bauteil liegt oder nicht. Auf diese Weise können die Randkanten der zu untersuchenden mindestens einen Lage exakt erfasst werden, so dass sich deren gegenseitige Ausrichtung überprüfen lässt.

Ein grundsätzliches Problem des mindestens einen Schwertes liegt darin, dass es nur in einem Bereich abgestützt werden kann, in dem die mindestens eine Lage noch nicht auf das mindestens eine Schwert gefaltet ist. Nur in diesem Bereich ist das mindestens eine Schwert von außen zugänglich. Andererseits muss das mindestens eine Schwert bis in einen Prüfbereich reichen, wobei sich zwischen der möglichen Befestigungsstelle des mindestens einen Schwertes und dem Prüfbereich wenigstens ein Faltwerk und gegebenenfalls ein Leimwerk befinden könnte. Da der mindestens eine Faltzuschnitt kontinuierlich weitertransportiert wird, ergibt sich in der Regel eine doch beträchtliche Schwertlänge. Zusätzlich ergibt sich das Problem, dass das mindestens eine Schwert keine allzu große Dicke haben darf, um den Faltzuschnitt nicht zu verformen und damit auch die Verbindung nicht zu beeinträchtigen. Unter diesen Nebenbedingungen ergibt sich in der Regel für das mindestens eine Schwert eine nicht in sich formsteife Ausbildung. Um das Schwert dennoch korrekt auszurichten, wird es von der mindestens einen Lage gestützt. Damit ist sichergestellt, dass das mindestens eine Schwert auch im Endbereich korrekt zum mindestens einen Faltzuschnitt ausgerichtet ist.

Für die eingesetzten Wellen haben sich sowohl akustische als auch elektromagnetische Wellen bewährt. Als akustische Wellen kommen insbesondere Ultraschallwellen in Betracht, da diese weniger auf Störgeräusche empfindlich sind als akustische Wellen im Hörbereich. Bei elektromagnetischen Wellen ist insbesondere der optische Bereich vom nahen Infrarot bis zum nahen UV vorzuziehen, da sich hierdurch ein besonders kompakter Aufbau bei gleichzeitig hoher Abtastgenauigkeit ergibt. Wichtig ist in jedem Fall, dass die eingesetzten Wellen tatsächlich von der Lage aus flächigem Material beeinflusst werden. Besteht das flächige Material beispielsweise aus völlig transparentem Kunststoff, so verbietet sich der Einsatz von sichtbarem Licht, es sei denn, man nutzt Reflektionseigenschaften des Materials. Ist das flächige Material dagegen sehr luftdurchlässig, so kann die akustische Erfassung der mindestens einen Lage problematisch werden. In diesem Fall sind elektromagnetische Wellen vorzuziehen.

Für die Wahl jener physikalischen Eigenschaft, die bezüglich des mindestens einen Schwertes unterschiedlich zum mindestens einen Faltzuschnitt ist, stehen je nach Aufbau der Prüfvorrichtung verschiedene Wege offen. Beispielsweise könnte die Reflektivität bezüglich der eingesetzten Wellen unterschiedlich sein. Damit wird erreicht, dass vom mindestens einen Bauteil ausgehende Wellen vom mindestens einen Faltzuschnitt einerseits und vom mindestens einen Schwert andererseits unterschiedlich reflektiert werden. Dabei spielt es grundsätzlich keine Rolle, ob dieser Unterschied in der Reflektivität von der Wellenlänge und/oder der Polarisation der Wellen abhängt. Ggf. kann der erzielte Kontrast in diesem Fall durch entsprechende Filter oder eine entsprechende Polarisierung der Wellen verbessert werden.

Alternativ oder zusätzlich kann diese physikalische Eigenschaft auch eine Polarisationsdrehung des reflektierten Lichtes im Verhältnis zum einfallenden Licht sein. In diesem Fall muss allerdings sichergestellt werden, dass die Wellen mit konkreter, vorgegebener Polarisation in Richtung des mindestens einen Schwertes gesendet werden, wobei das reflektierte Licht dann bezüglich seiner Polarisationseigenschaften ausgewertet wird. Damit kann detektiert werden, welche Bereiche des mindestens einen Schwertes von der mindestens einen Lage abgedeckt wurden.

Als weitere Alternative oder zusätzlich könnte die Emission der Wellen als unterschiedliche physikalische Eigenschaft genutzt werden. In diesem Fall würde das mindestens eine Schwert selbst die Wellen erzeugen und gegen das mindestens eine Bauteil richten. Dort, wo das mindestens eine Schwert von der mindestens einen Lage abgedeckt ist, werden die Wellen entsprechend gedämpft bis vollkommen abgeschattet. Diese Variante ist insbesondere bei bedruckten Materialien vorteilhaft, da auf diese Weise unabhängig von den gewählten Druckfarben und -mustern stets ein hoher Kontrast erzielbar ist.

Als weitere Alternative oder zusätzlich könnten die Wellen auch vom mindestens einen Schwert empfangen werden. In diesem Fall wäre das mindestens eine Schwert mit mindestens einem Detektor auszurüsten, welcher die vom mindestens einen Bauteil ausgesendeten Wellen empfängt. Auch in diesem Fall werden die Wellen von der mindestens einen Lage gedämpft, um den erforderlichen Kontrast zu liefern. Auch dieses Verfahren ist unabhängig von möglichen Bedruckungen des flächigen Materials.

Um exakte Daten über den Verlauf der zu prüfenden Kanten zu erhalten, werden die Wellen ortsaufgelöst erfasst. Dabei reicht es grundsätzlich aus, die Wellen quer zur Laufrichtung des mindestens einen Faltzuschnitts ortsaufgelöst zu erfassen. Bedingt durch den Transport des mindestens einen Faltzuschnitts kann eine Ortsauflösung in Laufrichtung beispielsweise durch wiederholtes Erfassen der Wellen in entsprechenden Zeitabständen erzielt werden. Die entsprechenden Messdaten lassen sich dann durch die bekannte Geschwindigkeit des mindestens einen Faltzuschnitts in ein zweidimensionales Bild umrechnen. Alternativ können die Wellen auch zweidimensional ortsaufgelöst erfasst werden, wodurch sich unmittelbar ein entsprechendes Bild ergibt. Unabhängig von der Wahl des Bilderfassungsverfahrens wird das gewonnene Bild anschließend einer Bildverarbeitung unterworfen. Im Rahmen dieser Bildverarbeitung kann das gewonnene Bild beispielsweise mit einem Soll-Bild verglichen werden. Abweichungen vom Sollbild zeigen dabei entsprechende Ausrichtungsfehler an. In diesem Fall wird vorzugsweise eine Schwelle definiert, ab der Abweichungen nicht mehr tolerierbar sind. Das Ergebnis der Bildverarbeitung ist in diesem Fall ein Digitalsignal, welches anzeigt, ob der in Lagen aufeinander gefaltete Faltzuschnitt innerhalb der Toleranzgrenzen liegt oder nicht. In der Regel wird der Bildvergleich aber nicht Pixel für Pixel vorgenommen, da dies zu einer sehr hohen Ausfallrate führen würde. Es werden vielmehr am Bild Binarisierungen, Verschiebungen und ggfs. Rotationen vorgenommen, um das gemessene Bild mit dem Soll-Bild in bestmögliche Übereinstimmung zu bringen. Erst nach diesen Operationen werden diese Bilder miteinander verglichen. Alternativ können aus dem gewonnenen Bild auch direkt die exakten Kantenlagen, Spaltbreiten, Spaltverläufe oder dgl. mit bekannten Bildverarbeitungsoperationen ermittelt werden. Beispielsweise geschieht dies durch Berechnung des Gradienten des Bildes und anschließende Schwellwert-Filterung. In diesem Fall kann ggf. auf ein Soll-Bild verzichtet und stattdessen die konkrete Ausrichtung der zu prüfenden Kanten aufeinander und/oder die Form von Spalten unmittelbar gemessen werden.

Für die Durchführung des Verfahrens hat sich eine Vorrichtung mit folgenden Merkmalen bewährt. Die erfindungsgemäße Vorrichtung dient zum Prüfen von mindestens einem aus flächigem Material gebildeten Faltzuschnitt. Dieser ist in einer Laufrichtung transportiert und quer dazu in Lagen gefaltet. Die Vorrichtung weist mindestens einen Emitter und mindestens einen Detektor für Wellen auf, wobei insbesondere an akustische und/oder elektromagnetische Wellen gedacht ist. Dabei ist es wichtig, dass diese Wellen durch die mindestens eine umgefaltete Lage des mindestens einen Faltzuschnitts hinreichend stark beeinflusst werden, so dass ein auswertbarer Kontrast zwischen der mindestens einen Lage einerseits und einem Bereich außerhalb dieser mindestens einen Lage andererseits entsteht. Zu diesem Zweck weist die Vorrichtung mindestens ein Schwert auf, welches zwischen den Lagen des mindestens einen Faltzuschnittes gehalten ist. Damit wird verhindert, dass eine nicht umgefaltete Lage des mindestens einen Faltzuschnitts den Messvorgang beeinträchtigt. Außerdem ist mindestens ein Bauteil vorgesehen, welches sich außerhalb der Lagen befindet. Zwischen dem mindestens einen Schwert einerseits und dem mindestens einen Bauteil andererseits ist die mindestens eine umgefaltete Lage des mindestens einen Faltzuschnitts vorgesehen, dessen Ausrichtung zu prüfen ist. Zu diesem Zweck sind im mindestens einen Schwert und/oder im mindestens einen Bauteil der mindestens eine Emitter und/oder der mindestens eine Detektor vorgesehen. Dabei können der mindestens eine Emitter und der mindestens eine Detektor gemeinsam im mindestens einen Bauteil oder im mindestens einen Schwert vorgesehen sein. Alternativ können der mindestens eine Emitter und der mindestens eine Detektor auch einerseits im mindestens einen Schwert und andererseits im mindestens einen Bauteil vorgesehen sein, wobei diese Zuordnung grundsätzlich beliebig ist. Wichtig ist lediglich, dass der mindestens eine Detektor die Wellen des mindestens einen Emitters nach Beeinflussung durch die mindestens eine Lage des mindestens einen Faltzuschnitts empfangen kann. Dabei ergibt sich entweder eine Reflex- oder eine Gabelanordnung. Zur Erzielung eines hinreichenden Kontrasts durch die mindestens eine Lage des mindestens einen Faltzuschnitts weist das mindestens eine Schwert mindestens eine der folgenden Eigenschaften auf:

Das mindestens eine Schwert kann den mindestens einen Emitter enthalten. In diesem Fall werden die Wellen vom mindestens einen Schwert abgegeben. Die mindestens eine Lage des mindestens einen Faltzuschnitts kann die Wellen dämpfen, so dass ein entsprechender Kontrast erzielt wird.

Alternativ oder zusätzlich kann das mindestens eine Schwert auch den mindestens einen Detektor enthalten. In diesem Fall werden die Wellen vom mindestens einen Schwert aufgenommen und in entsprechende elektrische Signale umgewandelt. Vorzugsweise befindet sich in diesem Fall der mindestens eine Emitter im mindestens einen Bauteil, so dass sich eine Gabelanordnung ergibt. Es ist aber auch daran gedacht, das mindestens eine Schwert sowohl mit dem mindestens einen Detektor als auch dem mindestens einen Emitter auszurüsten. In diesem Fall werden die Wellen vom mindestens einen Schwert sowohl ausgesendet als auch detektiert, so dass sich eine Reflexanordnung ergibt. Die Reflektion der Wellen erfolgt dabei vorzugsweise durch einen im mindestens einen Bauteil enthaltenen Reflektor, der die Wellen entsprechend reflektiert.

Zusätzlich könnte das Schwert auch eine Reflektivität für die Wellen aufweisen, die sich von der Reflektivität der Lage des Faltzuschnitts unterscheidet. In diesem Fall werden der mindestens eine Emitter und der mindestens eine Detektor vorzugsweise im mindestens einen Bauteil untergebracht, so dass sich eine entsprechende Reflektionsanordnung ergibt. Auf diese Weise ergibt sich ein besonders einfacher Aufbau des mindestens einen Schwertes, welches beispielsweise von einem dünnen Metallstreifen gebildet sein kann.

Zusätzlich könnte das Schwert auch die Polarisationsrichtung drehen, um den gewünschten Kontrast zu erzielen. In diesem Fall werden der mindestens eine Emitter und der mindestens eine Detektor vorzugsweise mit entsprechenden Polarisationsfiltern ausgerüstet. Alle diese Maßnahmen erlauben die Erzeugung eines optimalen Kontrasts, um die Form der mindestens einen Lage des mindestens einen Faltzuschnitts zu erfassen.

Für den mindestens einen Emitter kommt ein Ultraschallsender in Frage, welcher Ultraschallwellen aussendet. In diesem Fall ist der mindestens eine Detektor als Ultraschallempfänger ausgebildet. Diese Variante ist insbesondere für transparente Materialien vorzuziehen.

Im Falle von nicht transparenten Materialien wird als Emitter eine Lichtquelle und als Detektor ein Lichtdetektor vorgezogen. Diese Variante zeichnet sich durch einen kompakteren Aufbau und eine bessere Verfügbarkeit der erforderlichen Bauelemente aus.

Für die Lichtquelle hat sich insbesondere eine Glühlampe, eine Gasentladungsröhre, eine Leuchtdiode oder ein Laser bewährt. Insbesondere zur Erzielung eines sehr flachen Aufbaus des mindestens einen Schwertes wird Leuchtdioden der Vorzug gegeben. Insbesondere ist an Leuchtdioden aus organischem Material gedacht, die als flächige Kunststoffbahnen herstellbar sind.

Für den Lichtdetektor hat sich insbesondere eine Fotodiode, ein Fototransistor oder eine Kamera bewährt. Bei der Kamera ist insbesondere an Zeilen- und Flächenkameras gedacht. Die konkrete Übertragungstechnik der Kamera spielt dabei nur eine untergeordnete Rolle. Insbesondere ist an CCD- als auch an CMOS-Kameras gedacht. Diese Aufzählung ist allerdings nicht abschließend zu verstehen.

Um das mindestens eine Schwert besonders dünn ausbilden zu können, kann der mindestens einen Lichtquelle und/oder dem mindestens einen Lichtdetektor mindestens eine Faser, vorzugsweise eine Glas- und/oder Kunststofffaser zugeordnet sein. Diese Faser kann das Licht von der Lichtquelle wegführen oder dem Lichtdetektor zuführen. Durch diese Maßnahme kann die Lichtquelle bzw. der Lichtdetektor in einem Bereich des mindestens einen Schwertes angeordnet werden, in dem die Schwertdicke keine Rolle spielt. Dies ist beispielsweise ein Bereich, in dem der mindestens eine Faltzuschnitt noch nicht in Lagen aufeinander gefaltet ist. Die Fasern übernehmen dann den Lichttransport zum oder vom Prüfgebiet.

Zur Erzielung einer gleichmäßigen Ausleuchtung des Prüfgebiets, insbesondere bei punktförmigen Lichtquellen, ist im mindestens einen Schwert mindestens eine Streuscheibe vorgesehen. Diese sorgt insbesondere für die Erzeugung von diffusem Licht, welches unerwünschte Glanzreflektion am Faltzuschnitt vermeidet.

Um aus den gewonnenen Bilddaten des Detektors eine Aussage bezüglich des Prüfergebnisses zu erzielen, ist diesem mindestens eine Bildverarbeitungsvorrichtung nachgeordnet. Diese erzeugt aus den Bilddaten ein Entscheidungskriterium, inwieweit der erstellte mindestens eine Faltzuschnitt den Anforderungen entspricht. Dieses Entscheidungskriterium kann dazu genutzt werden, schlecht gefaltete Faltzuschnitte auszusondern, zu markieren oder ein Alarmsignal zu erzeugen.

Der Erfindungsgegenstand sowie das erfindungsgemäße Verfahren werden beispielhaft anhand der Zeichnung erläutert, ohne den Schutzumfang zu beschränken.

Es zeigt:
- Figur 1: eine räumliche Prinzipdarstellung einer Vorrichtung zum Prüfen mindestens eines Faltzuschnittes,
- Figur 2: eine Schnittdarstellung durch die Vorrichtung gemäß Figur 1,
- Figur 3: eine alternative Ausführungsform der Vorrichtung gemäß Figur 2,
- Figur 4: eine weitere alternative Ausführungsform gemäß Figur 2,
- Figur 5: eine Ausführungsform mit im Schwert integriertem Detektor,
- Figur 6: eine Ausführungsform mit im Schwert integriertem Detektor und Emitter,
- Figur 7: eine Schnittdarstellung eines Schwertes mit Faser und
- Figur 8: eine alternative Ausführungsform des Schwertes gemäß Figur 7 mit Faser.
- Figur 9: eine Detaildarstellung eines ordnungsgemäß gefalteten Faltzuschnitts,
- Figur 10: die Darstellung gemäß Figur 9 mit zu engem Spalt,
- Figur 11: die Darstellung gemäß Figur 9 mit zu weitem Spalt und
- Figur 12: die Darstellung gemäß Figur 9 mit einem winkelig verzerrtem Spalt.

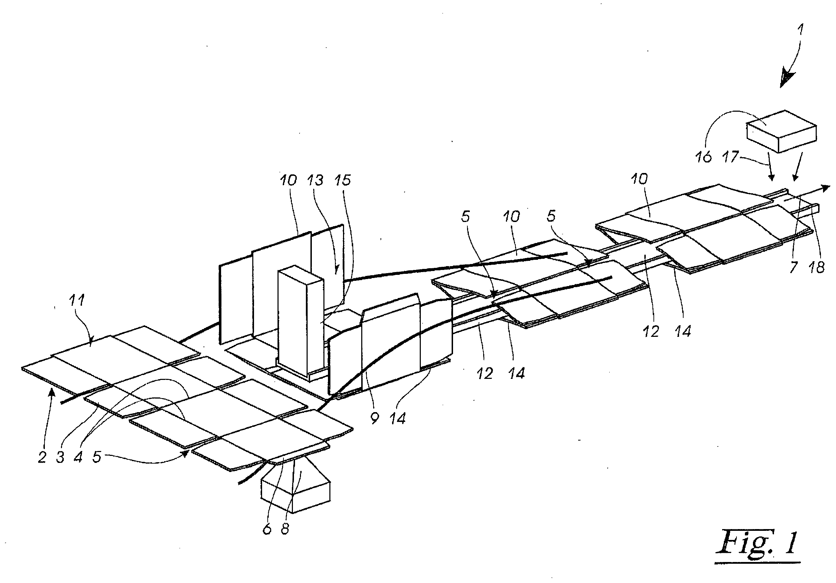

Die Figur 1 zeigt eine Vorrichtung 1 zum Prüfen eines Faltzuschnitts 2, bestehend aus einem flächigen Material 3, insbesondere Karton oder Wellpappe. In der Regel zeigen die Faltzuschnitte 2 nach unten, so dass die Figur 1 die Vorrichtung 1 von unten zeigt. Es gibt jedoch auch Einbausituationen, bei denen die Faltzuschnitte 2 nach oben zeigen, so dass die Figur 1 die Vorrichtung 1 von oben zeigt. Die Darstellung gemäß Figur 1 ist dabei eine reine Prinzipdarstellung, wobei auf die Darstellung aller für das Verständnis des Erfindungsgegenstandes unwesentlicher Komponenten verzichtet wurde.

Der Faltzuschnitt 2 weist mehrere Faltkanten 4 auf, die beispielsweise durch Rillen oder Stauchen in das flächige Material 3 eingeformt sind. Außerdem sind im Faltzuschnitt 2 Spalten 5 ausgeformt, welche das ungehinderte Zusammenfalten des Faltzuschnitts 2 zu einer quaderförmigen Verpackung erlauben. Diese Spalten sind idealerweise so dimensioniert, dass Boden- bzw. Deckellaschen gerade noch im Inneren des quaderförmig zusammengestellten Faltzuschnitts 2 Platz haben. Auf diese Weise ist zum einen sichergestellt, dass der Faltzuschnitt 2 quaderförmig gefaltet werden kann, zum anderen stabilisieren die Boden- und Deckellaschen die Seitenwände des Faltzuschnitts 2 und sorgen auf diese Weise für eine beträchtliche Erhöhung der Stabilität. Die Dimensionierung dieser Spalte 5 ergibt sich größtenteils durch die gewählte Stanzform.

Der Faltzuschnitt 2 weist außerdem eine Klebelasche 6 auf, um den Faltzuschnitt 2 zu einer mantelartigen Konfiguration umformen zu können. Jene Spalten 5, die sich im Bereich der Klebelasche 6 befinden, sind damit in ihrer Form und Breite nicht mehr allein durch das Stanzwerkzeug, sondern auch durch die Faltung und Verklebung bedingt. Je größer die Überlappung im Klebebereich ist, um so schmaler wird der Spalt 5 in diesem Bereich ausgebildet werden. Bei einer nicht exakt parallelen Faltung ergeben sich zusätzlich Winkelverzerrungen des Spaltes 5.

Der Faltzuschnitt 2 wird in Laufrichtung 7 kontinuierlich transportiert, wobei die einzelnen Faltzuschnitte 2 sequentiell hintereinander folgen. In der Figur 1 ist ein Leimwerk 8 angedeutet, welches die Klebelasche 6 mit einem entsprechenden Klebstoff bestreicht. Diesem Leimwerk ist ein Faltwerk 9 nachgeordnet, welches eine Lage 10 des Faltzuschnittes 2 umfaltet. Dabei kommt die Klebelasche 6 mit einem dieser gegenüberliegenden Endbereich 11 des Faltzuschnittes 2 in flächigen Kontakt und sorgt auf diese Weise für eine dauerhafte Verbindung miteinander.

Um die korrekte Faltung des Faltzuschnittes 2 überprüfen zu können, wird mindestens eine der Spalten 5 im Bereich der Klebelasche 6 analysiert. Zu diesem Zweck weist die Vorrichtung 1 ein Schwert 12 auf. Dieses Schwert 12 erstreckt sich im Wesentlichen längs der Laufrichtung 7 des Faltzuschnittes 2. Es ist ausschließlich in einem Bereich 13 abgestützt, in dem der Faltzuschnitt 2 noch nicht durch das Faltwerk 9 vollständig umgefaltet ist. Damit liegt im Bereich 13 eine nicht umzufaltende Lage 14 und eine umzufaltende Lage 10 in offener Konfiguration vor, so dass hier Platz für eine entsprechende Stützkonstruktion 15 des Schwertes 12 ist.

Dem Schwert 12 liegt ein Bauteil 16 gegenüber. Zwischen dem Schwert 12 und dem Bauteil 16 breiten sich Wellen 17 aus, die zur Prüfung des Faltzuschnitts 2 eingesetzt werden. Diese Prüfung erstreckt sich auf mindestens einen jener Spalte 5, die im Bereich der Klebelasche 6 vorgesehen sind. Alle anderen Spalte 5 sind in ihrer Geometrie durch ein Stanzwerkzeug vorgegeben und damit fehlerfrei, so dass deren Analyse entfallen kann.

Um zu verhindern, dass das Schwert 12 die Klebelasche 6 erfasst und damit die Ausrichtung der Lagen 10, 14 zueinander beeinträchtigt, weist das Schwert 12 Kufen 18 auf. Diese sind beidseits des Schwertes 12 vorgesehen und garantieren, dass das Schwert 12 nicht die Klebelasche 6 berührt.

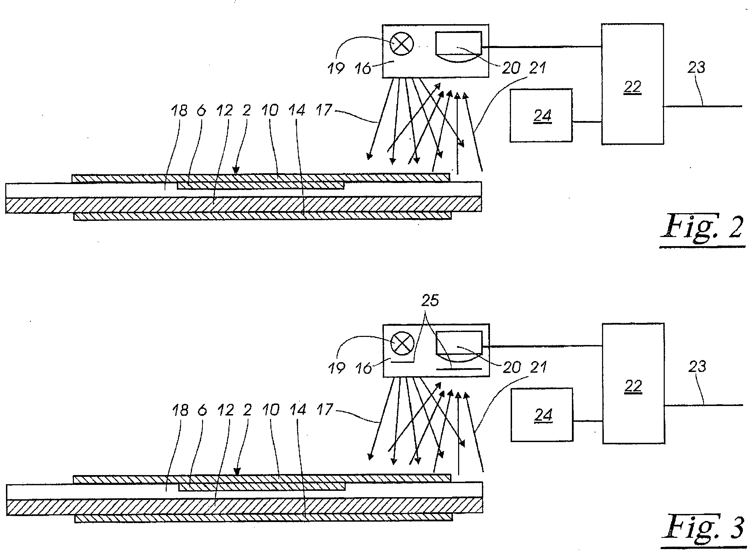

Der genaue Aufbau des Schwertes 12 und des Bauteils 16 erfolgen anhand der Schnittdarstellung gemäß Figur 2. Man erkennt aus dieser Schnittdarstellung, dass das Schwert 12 zwischen die Lagen 10, 14 des Faltzuschnitts 2 greift. Das Schwert 12 verhindert auf diese Weise, dass die Erfassung der umgefalteten und geklebten Lage 10 durch die Lage 14 gestört wird.

Im Bauteil 16 sind ein Emitter 19 und ein Detektor 20 vorgesehen. Der Emitter 19 sendet die Wellen 17 aus, die zum Teil auf die Lage 10 des Faltzuschnittes 2 und zum Teil direkt auf das Schwert 12 fallen. Das Schwert 12 ist dabei derart ausgebildet, dass es die Wellen 17 entweder gut reflektiert oder gut absorbiert. Damit ergibt sich zwischen jenen Bereichen, in denen sich die Lage 10 des Faltzuschnittes 2 befindet und allen anderen Bereichen ein starker Kontrast in den reflektierten Wellen 21, die vom Detektor 20 ortsaufgelöst erfasst werden.

Dem Detektor 20 ist eine Bildverarbeitungsvorrichtung 22 nachgeordnet, die über einen Signalweg 23 ein Signal abgibt, welches anzeigt, ob sich der Faltzuschnitt 2 innerhalb des erlaubten Toleranzbereichs befindet. Dabei ist es insbesondere wichtig, einen Versatz der Lage 10 in und/oder quer zur Laufrichtung 7 zu erfassen. Zu diesem Zweck vergleicht die Bildverarbeitungsvorrichtung 22 das vom Detektor 20 aufgenommene Bild mit einem Soll-Bild 24 und gibt bei Überschreitung einer vorgebbaren Fehlerschwelle ein Fehlersignal am Signalweg 23 aus. Dieses Fehlersignal kann beispielsweise dazu genutzt werden, den fehlerhaften Faltzuschnitt zu markieren, auszusondern oder einen Alarm auszulösen.

Die Figur 3 zeigt eine alternative Ausführungsform, wobei gleiche Bezugszeichen gleiche Teile benennen. Im Folgenden wird lediglich auf die Unterschiede zur Ausführungsform gemäß Figur 2 eingegangen. Bei der Ausführungsform gemäß Figur 3 ist das Schwert 12 derart ausgebildet, dass es die Polarisationsrichtung der Wellen 17 während der Reflektion dreht. Außerdem sind vor dem Detektor 20 und hinter dem Emitter 19 Polarisationsfilter 25 vorgesehen. Auf diese Weise kann die Drehung der Polarisationsrichtung durch das Schwert 12 erfasst werden. Der Faltzuschnitt 2 besteht dabei aus einem Material 3, welches die Polarisationsrichtung der Wellen 17 nicht oder in anderer Weise dreht, so dass auf diese Weise - bedingt durch die Polarisationsfilter 25 - ein entsprechender Kontrast geschaffen wird. Dieser Kontrast erlaubt die exakte Erfassung der Lage 10.

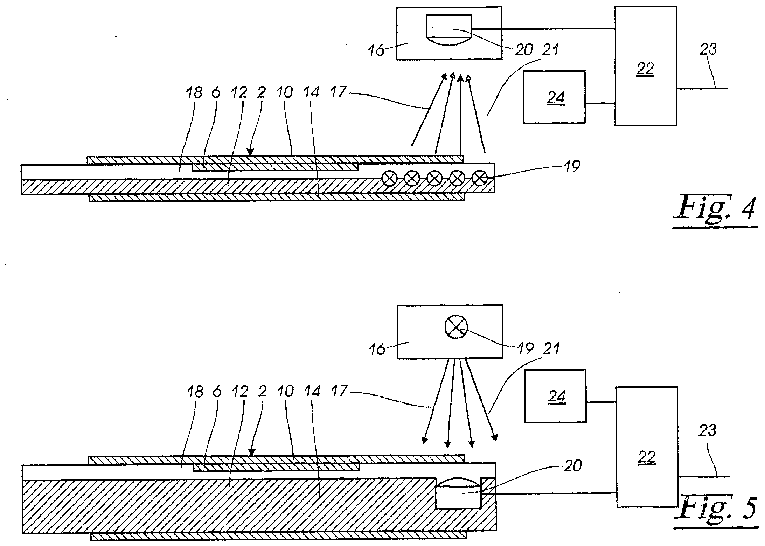

Die Figur 4 zeigt eine weitere alternative Ausführungsform. Bei dieser ist der Emitter 19 nicht im Bauteil 16, sondern im Schwert 12 vorgesehen. Hierdurch ergibt sich eine Gabelanordnung, wobei die Wellen 17 vom Schwert 12 zum Bauteil 16 laufen. Da das Material 3 des Faltzuschnittes 2 grundsätzlich keine Wellen 17 aussenden kann, ergibt sich auf diese Weise - unabhängig von eventuellen Bedruckungen des Faltzuschnittes 2 - ein hoher Kontrast.

Die Figur 5 zeigt eine weitere alternative Ausführungsform. Bei dieser sind im Gegensatz zur Ausführungsform gemäß Figur 4 Emitter 19 und Detektor 20 vertauscht. Der Detektor 20 befindet sich dabei im Schwert 12, während sich der Emitter 19 im Bauteil 16 befindet. Hierbei ergeben sich grundsätzlich die gleichen Vorteile wie bei der Ausführungsform gemäß Figur 4. Allerdings ist das Schwert 12 - bedingt durch die Unterbringung des Detektors 20 - in der Regel dicker auszubilden. Dies spielt jedoch bei großen Faltzuschnitten in der Regel keine Rolle.

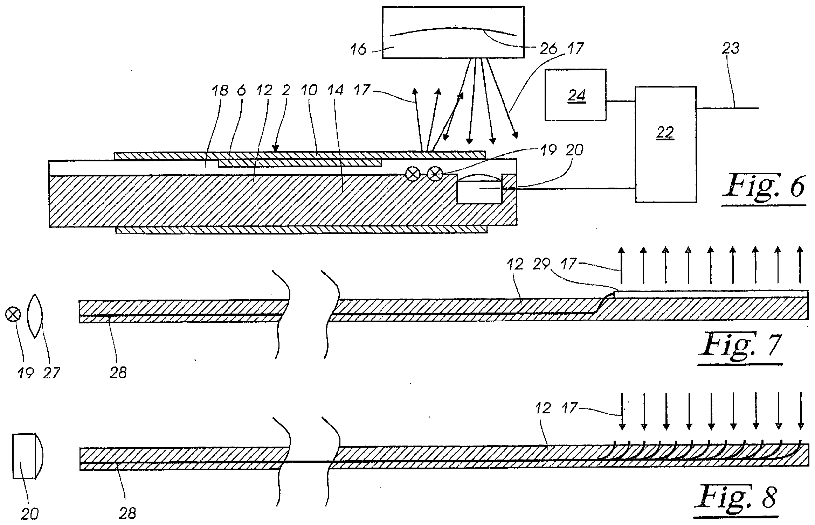

Die Figur 6 zeigt eine weitere alternative Ausführungsform, bei der Emitter 19 und Detektor 20 gemeinsam im Schwert 12 angeordnet sind. Im Bauteil 16 ist in diesem Fall ein Reflektor 26 angeordnet, der die Wellen 17 des Emitters 19 auf den Detektor 20 reflektiert.

Die Figur 7 zeigt eine alternative Ausführungsform des Schwertes 12 gemäß Figur 4. Dabei ist der Emitter 19 im Bereich 13 der Stützkonstruktion 15 vorgesehen. Die Wellen 17 werden mittels einer Linse 27 in eine Faser 28 eingekoppelt. Diese ist vorzugsweise als Glas- oder Kunststofffaser ausgebildet. Die Faser 28 steht mit einer Streuscheibe 29 in Verbindung, die die Wellen 17 aus dem Schwert 12 herausführen. Auf diese Weise kann das Schwert 12 besonders dünn ausgebildet sein, ohne entsprechende Beschränkungen bei der Ausbildung des Emitters 19 hinnehmen zu müssen.

Schließlich zeigt die Figur 8 eine weitere alternative Ausführungsform des Schwertes 12. Das Schwert 12 enthält ein Bündel von Fasern 28, die mit dem Detektor 20 gekoppelt sind. Der Detektor 20 ist in diesem Fall im Bereich 13 der Stützkonstruktion 15 vorgesehen, so dass dessen Baugröße die Dicke des Schwertes 12 nicht zu groß werden lässt.

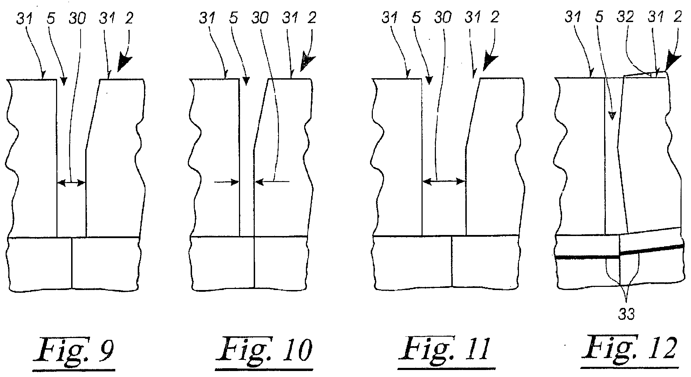

Die Figur 9 zeigt eine Detaildarstellung eines Faltzuschnitts 2 mit ordnungsgemäßer Verklebung. Der Spalt 5 weist eine Breite 30 auf, die mit hinreichender Genauigkeit dem Sollwert entspricht. Stirnkanten 31 fluchten miteinander, so dass der Spalt 5 auch die korrekte Form besitzt. Dieser Faltzuschnitt 2 enthält somit keinerlei Beanstandungen und die Vorrichtung 1 löst damit auch keinerlei Fehlersignal aus.

Die Figur 10 zeigt die Darstellung gemäß Figur 9, wobei der Spalt 5 in seiner Breite 30 zu eng ausgebildet ist. Dies ist eine Folge einer zu großen Überlappung im Bereich der Klebelasche 6. Dieser Fehler führt in der Regel dazu, dass der Faltzuschnitt 2 nicht mehr in die gewünschte quaderförmige Gestalt gefaltet werden kann, da die Bodenlasche an den Seitenwänden beim Zusammenfalten anstößt. Für diesen Faltzuschnitt 2 gibt die Vorrichtung 1 ein entsprechendes Fehlersignal aus.

Die Figur 11 zeigt einen weiteren Faltzuschnitt 2 mit zu weitem Spalt 5. Die Spaltbreite 30 überschreitet daher einen bestimmten Toleranzbereich. Ein derartiger Faltzuschnitt 2 könnte zwar problemlos zusammengebaut werden, die Bodenlasche liegt jedoch nicht mehr an den Seitenwänden an, so dass sie die Seitenwände auch nicht mehr stabilisieren kann. Ein derartiger Faltzuschnitt 2 besitzt daher nicht die erforderliche Stabilität und wird daher ebenfalls von der Vorrichtung 1 ausgesondert.

Schließlich zeigt die Figur 12 einen weiteren Faltzuschnitt 2. Bei diesem fluchten die Kanten 31 nicht mehr miteinander, sondern sie stehen vielmehr in einem Winkel 32 zueinander. Dieser Winkel 32 entsteht durch einen Versatz in Laufrichtung 7. Dieser Fehler führt einerseits zu Problemen mit dem Zusammenbau des Faltzuschnitts 2 und andererseits zu einem Versatz in einem Druckbild 33. Dieser Versatz ist insbesondere optisch wenig ansprechend. Außerdem ergibt sich durch die oben beschriebenen Fehler eine verringerte Stabilität des Faltzuschnitts 2. Dieser wird demnach ebenfalls ausgesondert.

**Bezugszeichenliste**

| | | | |
|---|---|---|---|
| 1 | Vorrichtung | 18 | Kufe |
| 2 | Faltzuschnitt | 19 | Emitter |
| 3 | Material | 20 | Detektor |
| 4 | Faltkante | 21 | Welle |
| 5 | Spalt | 22 | Bildverarbeitungsvorrichtung |
| 6 | Klebelasche | 23 | Signalweg |
| 7 | Laufrichtung | 24 | Soll-Bild |
| 8 | Leimwerk | 25 | Polarisationsfilter |
| 9 | Faltwerk | 26 | Reflektor |
| 10 | Lage | 27 | Linse |
| 11 | Endbereich | 28 | Faser |
| 12 | Schwert | 29 | Streuscheibe |
| 13 | Bereich | 30 | Breite |
| 14 | Lage | 31 | Stirnkante |
| 15 | Stützkonstruktion | 32 | Winkel |
| 16 | Bauteil | 33 | Druckbild |
| 17 | Welle | | |

## Patentansprüche

1. Verfahren zum Prüfen von mindestens einem aus flächigem Material (3) gebildeten Faltzuschnitt (2), welcher in einer Laufrichtung (7) transportiert und quer dazu in Lagen (10, 14) aufeinander gefaltet wird, wobei der mindestens eine Faltzuschnitt (2) an mindestens einem Schwert (12) entlang geführt wird, auf das mindestens eine der Lagen (10) gefaltet wird, so dass sich das mindestens eine Schwert (12) zwischen den Lagen (10, 14) befindet, und dem mindestens einen Schwert (12) mindestens ein Bauteil (16) gegenüber liegt, welches außerhalb des mindestens einen Faltzuschnitts (2) vorgesehen wird und zwischen dem mindestens einen Schwert (12) und dem mindestens einen Bauteil (16) Wellen (17, 21) propagiert werden, wobei das mindestens eine Schwert (12) bezüglich der Wellen (17, 21) mindestens eine physikalische Eigenschaft aufweist, die sich von der des mindestens einen Faltzuschnitts (2) unterscheidet, **dadurch gekennzeichnet, dass** das mindestens eine Schwert (12) die Wellen (17, 21) emittiert und/oder detektiert.

2. Verfahren nach Anspruch 1 oder nach dem Oberbegriff des Anspruchs 1, **dadurch gekennzeichnet, dass** das mindestens eine Schwert (12) von mindestens einer der Lagen (10, 14) gestützt ausgebildet wird.

3. Verfahren nach Anspruch 1 oder 2, **dadurch gekennzeichnet, dass** die Wellen (17, 21) akustische und/oder elektromagnetische Wellen sind.

4. Verfahren nach mindestens einem der Ansprüche 1 bis 3, **dadurch gekennzeichnet, dass** die Wellen (17) ortsaufgelöst erfasst und einer Bildverarbeitung unterworfen werden.

5. Vorrichtung zum Prüfen von mindestens einem aus flächigem Material (3) gebildeten Faltzuschnitt (2), welcher in einer Laufrichtung (7) transportiert und quer dazu in Lagen (10, 14) gefaltet ist, wobei die Vorrichtung (1) mindestens einen Emitter (19) und mindestens einen Detektor (20) für Wellen (17, 21) aufweist, **dadurch gekennzeichnet, dass** die Vorrichtung (1) mindestens ein Schwert (12) aufweist, welches zwischen den Lagen (10, 14) gehalten ist, und die Vorrichtung (1) mindestens ein Bauteil (16) aufweist, welches außerhalb der Lagen (10, 14) vorgesehen ist, wobei im mindestens einen Schwert (12) und/oder im mindestens einen Bauteil (16) der mindestens eine Emitter (19) und der mindestens eine Detektor (20) für die Wellen (17, 21) vorgesehen ist, wobei der mindestens eine Detektor (20) die Wellen (17, 21) des mindestens einen Emitters (19), beeinflusst durch die mindestens eine Lage (10) des mindestens einen Faltzuschnitts (2), empfangen kann, und das mindestens eine Schwert (12) mindestens eine der folgenden Eigenschaften aufweist:
a. Es enthält den mindestens einen Emitter (19),
b. es enthält den mindestens einen Detektor (20).

6. Vorrichtung nach Anspruch 5, **dadurch gekennzeichnet, dass** der mindestens eine Emitter (19) ein Ultraschallsender und der mindestens eine Detektor (20) ein Ultraschallempfänger ist.

7. Vorrichtung nach Anspruch 5, **dadurch gekennzeichnet, dass** der mindestens eine Emitter (19) eine Lichtquelle und der mindestens eine Detektor (20) ein Lichtdetektor ist.

8. Vorrichtung nach Anspruch 7, **dadurch gekennzeichnet, dass** die Lichtquelle eine Glühlampe, Gasentladungsröhre, Leuchtdiode oder ein Laser ist.

9. Vorrichtung nach Anspruch 7 oder 8, **dadurch gekennzeichnet, dass** der Lichtdetektor eine Fotodiode, ein Fototransistor oder eine Kamera ist.

10. Vorrichtung nach mindestens einem der Ansprüche 7 bis 9, **dadurch gekennzeichnet, dass** der Lichtquelle und/oder dem Lichtdetektor mindestens eine Faser (28) zugeordnet ist, die Licht von der Lichtquelle weg und/oder zum Lichtdetektor leiten kann.

11. Vorrichtung nach mindestens einem der Ansprüche 7 bis 10, **dadurch gekennzeichnet, dass** im mindestens einen Schwert (12) die Lichtquelle und mindestens eine Streuscheibe (29) vorgesehen sind.

12. Vorrichtung nach mindestens einem der Ansprüche 5 bis 11, **dadurch gekennzeichnet, dass** dem mindestens einen Detektor (20) mindestens eine Bildverarbeitungsvorrichtung (22) nachgeordnet ist.

## Claims

1. Method for checking at least one folded blank (2) which is formed from a planar material (3) and is transported in a running direction (7) and folded transversely thereto into layers (10, 14), wherein the at least one folded blank (2) is guided along at least one blade (12) onto which at least one of the layers (10) is folded, with the result that the at least one blade (12) is located between the layers (10, 14) and at least one component (16) which is provided outside the at least one folded blank (2) lies opposite the at least one blade (12), and waves (17, 21) are propagated between the at least one blade (12) and the at least one component (16), wherein the at least one blade (12) has, with respect to the waves (17, 21), at least one physical property which differs from that of the at least one folded blank (2) **characterized in that** the at least one blade (12) emits and/or detects the waves (17, 21).

2. Method according to Claim 1 or according to the preamble of Claim 1, **characterized in that** the at least one blade (12) is formed under the support of at least one of the layers (10, 14).

3. Method according to Claim 1 or 2, **characterized in that** the waves (17, 21) are acoustic and/or electromagnetic waves.

4. Method according to at least one of Claims 1 to 3, **characterized in that** the waves (17) are detected with local resolution and subjected to image processing.

5. Device for checking at least one folded blank (2) which is formed from planar material (3), transported in a running direction (7) and folded transversely with respect thereto into layers (10, 14), wherein the device (1) has at least one emitter (19) and at least one detector (20) for waves (17, 21), **characterized in that** the device (1) has at least one blade (12) which is secured between the layers (10, 14), and the device (1) has at least one component (16) which is provided outside the layers (10, 14), wherein the at least one emitter (19) and the at least one detector (20) for the waves (17, 21) are/is provided in the at least one blade (12) and/or in the at least one component (16), wherein the at least one detector (20) can receive the waves (17, 21) of the at least one emitter (19) under the influence of the at least one layer (10) of the at least one folded blank (2), and the at least one blade (12) has at least one of the following properties:
a. it contains the at least one emitter (19),
b. it contains the at least one detector (20).

6. Device according to Claim 5, **characterized in that** the at least one emitter (19) is an ultrasonic transmitter and the at least one detector (20) is an ultrasonic receiver.

7. Device according to Claim 5, **characterized in that** the at least one emitter (19) is a light source, and the at least one detector (20) is a light detector.

8. Device according to Claim 7, **characterized in that** the light source is an incandescent lamp, a gas discharge tube, a light-emitting diode or a laser.

9. Device according to Claim 7 or 8, **characterized in that** the light detector is a photodiode, a phototransistor or a camera.

10. Device according to at least one of Claims 7 to 9, **characterized in that** a fibre (28) which can guide light away from the light source and/or to the light detector is assigned to the light source and/or to the light detector.

11. Device according to at least one of Claims 7 to 10, **characterized in that** the light source and at least one lens (29) are provided in the at least one blade (12).

12. Device according to at least one of Claims 5 to 11, **characterized in that** at least one image-processing device (22) is arranged downstream of the at least one detector (20).

## Revendications

1. Procédé de contrôle d'au moins une découpe de pliage (2) formée à partir d'un matériau plat (3), qui est transporté dans une direction de déplacement (7) et est plié sur lui-même transversalement à ladite direction dans des couches (10, 14), dans lequel l'au moins une découpe de pliage (2) est guidée le long d'au moins une barre de guidage (12), est pliée sur au moins l'une des couches (10) de manière à ce que l'au moins une barre de guidage (12) se trouve entre les couches (10, 14), et au moins un composant (16) se situe à l'opposé d'au moins une barre de guidage (12), lequel composant est prévu à l'extérieur de l'au moins une découpe de pliage (2), et des ondes (17, 21) sont amenées à se propager entre l'au moins une barre de guidage (12) et l'au moins un composant (16), dans lequel l'au moins une barre de guidage (12) présente au moins une propriété physique par rapport aux ondes (17, 21), qui est différente de celle de l'au moins une découpe de pliage (2), **caractérisé en ce que** l'au moins une barre de guidage (12) émet et/ou détecte les ondes (17, 21) .

2. Procédé selon la revendication 1 ou selon le préambule de la revendication 1, **caractérisé en ce que** l'au moins une barre de guidage (12) est réalisée de manière renforcée par au moins l'une des couches (10, 14) .

3. Procédé selon la revendication 1 ou 2, **caractérisé en ce que** les ondes (17, 21) sont des ondes acoustiques et/ou électromagnétiques.

4. Procédé selon l'une quelconque des revendications 1 à 3, **caractérisé en ce que** les ondes (17) sont détectées de manière résolue spatialement et sont soumises à un traitement d'image.

5. Dispositif de contrôle d'au moins une découpe de pliage (2) formée à partir d'un matériau plat (3), laquelle découpe de pliage est transportée dans une direction de déplacement (7) et est pliée sur elle-même transversalement à ladite direction dans des couches (10, 14), dans lequel le dispositif (1) comprend au moins un émetteur (19) et au moins un détecteur (20) des ondes (17, 21), **caractérisé en ce que** le dispositif (1) comporte au moins une barre de guidage (12) qui est maintenue entre les couches (10, 14), et **en ce que** le dispositif (1) comprend au moins un composant (16) qui est prévu à l'extérieur des couches (10, 14), dans lequel l'au moins un émetteur (19) et l'au moins un détecteur (20) des ondes (17, 21) sont prévus dans au moins une barre de guidage (12) et/ou dans au moins un composant (16), dans lequel l'au moins un détecteur (20) peut recevoir les ondes (17, 21) de l'au moins un émetteur (19), de manière influencée par l'au moins une couche (10) de l'au moins une découpe de pliage (2), et **en ce que** l'au moins une barre de guidage (12) présente l'au moins une des propriétés suivantes :
a. elle contient l'au moins un émetteur (19),
b. elle contient l'au moins un détecteur (20).

6. Dispositif selon la revendication 5, **caractérisé en ce que** l'au moins un émetteur (19) est un émetteur à ultrasons et **en ce que** l'au moins un détecteur (20) est un détecteur à ultrasons.

7. Dispositif selon la revendication 5, **caractérisé en ce que** l'au moins un émetteur (19) est une source de lumière et **en ce que** l'au moins un détecteur (20) est un détecteur de lumière.

8. Dispositif selon la revendication 7, **caractérisé en ce que** la source de lumière est une lampe à incandescence, un tube à décharge de gaz, une diode électroluminescente ou un laser.

9. Dispositif selon la revendication 7 oui 8, **caractérisé en ce que** le détecteur de lumière est une photodiode, un phototransistor ou une caméra.

10. Dispositif selon au moins l'une des revendications 7 à 9, **caractérisé en ce qu'**une fibre (28) qui peut guider la lumière depuis la source de lumière et/ou vers le détecteur de lumière est associée à la source de lumière et/ou au détecteur de lumière.

11. Dispositif selon au moins l'une des revendications 7 à 10, **caractérisé en ce que** la source de lumière et au moins un disque diffuseur (29) sont prévus dans l'au moins une barre de guidage (12).

12. Dispositif selon au moins l'une des revendications 5 à 11, **caractérisé en ce qu'**au moins un dispositif de traitement d'image (22) est disposé en aval de l'au moins un détecteur (20).
